# EUROPEAN PATENT SPECIFICATION

(11) **EP 2 873 411 B1**
(45) Date of publication and mention of the grant of the patent: **14.10.2020**
(21) Application number: 14193071.9
(22) Date of filing: 13.11.2014
(51) Int. Cl.: A61K 8/24, A61Q 5/06, A61Q 5/10, A61K 8/365, A61K 8/86

(54) **Additive for hair dye**
Additiv für Haarfärbemittel
Additif pour colorant capillaire

(30) Priority: 15.11.2013 IT MI20131900
(43) Date of publication of application: 20.05.2015
(73) Proprietor: Giannico, Paolo, 38060 Villa Lagarina (IT); Arcelli, Antonio, 40141 Bologna (IT); Danielli, Marco, 40133 Bologna (IT)
(72) Inventor: GIANNICO, Paolo, 38060 Villa Lagarina (IT); ARCELLI, Antonio, 40141 Bologna (IT)
(74) Representative: Lunati & Mazzoni S.r.L.

(56) References cited:
- EP-A1- 2 277 498
- WO-A2-2006/106390
- WO-A2-2008/020983
- JP-A- 2007 262 001
- US-A1- 2007 169 285
- DATABASE GNPD [Online] MINTEL; 25 February 2014 (2014-02-25), anonymous: "Hair Colouring Mousse", XP055684359, retrieved from www.gnpd.com Database accession no. 2329927

## Description

The present invention relates to an additive for hair dye of the type as recited in the preamble of the first claim.

As known, hair dyes suitable to enhance the natural colour, lighten the shade, or return grey hair to its original colour are widely used.

In particular, among the most commonly used hair dyes are oxidation dyes which lighten or darken the hair colour, intensify or alter the reflections, ensuring a durable and lasting result.

These dyes are generally produced in the form of cream, oily or gelatinous products containing colourless substances, known as precursors, which are transformed into dyes only after oxidation, i.e. only after mixing with an oxidising agent.

Usually, the oxidising agent used is hydrogen peroxide H₂O₂ which, in addition to participating in the formation of pigments, facilitates the colouring process by exercising a bleaching action on the hair, i.e. it destroys the melanin present in hair which thus becomes lighter in colour and therefore more subject to dyeing.

The precursors are organic compounds belonging to the benzene series in which amino - NH₂ or alcohol - OH groups are present, such as para-diaminobenzene, meta-dihydroxybenzene and para-aminophenol.

The known technique cited above has some significant drawbacks.

One important drawback is represented by the fumes of hair dyes.

In fact, the mixing of the oxidising agent and the precursors leads to the development of ammonia which, vaporising almost instantly, leads to the formation of an especially irritating and malodorous gas/vapour.

This problem is particularly evident when one considers that this mixing is done by the hairdresser in situ and only at the time of performing dyeing so that these fumes are inhaled in large part by the hairdresser and by the customers.

Furthermore, the compound obtained by said mixing is left on the hair for about 30-40 minutes, thus forcing customer to inhale large quantities of ammonia.

In this situation the technical purpose of the present invention is to devise an additive for hair dyes able to substantially overcome the drawbacks mentioned above. Within the sphere of said technical purpose one important aim of the invention is to obtain a hair dye additive which is able to reduce the exhalation of ammonia by hair dyes.

Another important purpose of the invention is thus to obtain an additive which makes it possible to limit and, in particular, to abate the harmfulness of hair dyes.

One important purpose is moreover identifiable in the need to obtain an additive which can be added to a hair dye without changing the colour and, therefore, without altering the dyeing of the hair. There have already been attempts in the prior art to reduce the ammonia odour, e.g. in WO2006/106390. In the present disclosure, the
technical purpose and specified aims are achieved by an additive for hair dye as claimed in the appended Claim 1.

Preferred embodiments are evident from the dependent claims.

The additive for hair dye according to the invention relates to a particular compound to be added and thus mixed with the hair dye at the time of use thereof and, more precisely, to be added to the dye at the moment of mixing the precursors and the oxidising agent.

It mainly comprises citric acid suitable to combine with ammonia to form a soluble salt; phosphoric acid suitable to combine with the ammonia to form a soluble salt; and PEG, i.e. polyethylene glycol which acts as a thickener and viscosing agent, appropriately PEG 200.

In particular, the content in percentage in weight in relation to the additive 1 is comprised between 30% and 50% for the citric acid, between 30% and 50% for the phosphoric acid (85%) and between 20% and 30% for the PEG 200.

Additionally, the additive may include at least one and, in detail, at least two of the following: dimethicone meadowfoamate, dimethicone PEG-8 meadowfoamate suitable to ensure greater solubility of the additive and better applicability of the dye to the hair, propylene glycol suitable to control the viscosity and correct emulsification of the components of the dye, and water, preferably deionized and also suitable to promote the penetration of water by counteracting any drying effect of the dye, etidronic acid, constituting a chelating medium.

Appropriately, the content as a percentage in weight in relation to the additive 1 of each of these substances is substantially less than 6%.

In particular, the content in percentage in weight in relation to the additive 1 is substantially between 2% and 5% for the dimethicone meadowfoamate and, more specifically, substantially between 3% and 4%.

The content in percentage in weight in relation to the additive 1 is substantially between 2% and 5% for the dimethicone PEG-8 meadowfoamate and, in detail, substantially between 2% and 3.7%.

The content in percentage in weight in relation to the additive 1 is substantially between 2% and 6% for the propylene glycol and, preferably, substantially between 3% and 5%.

The content in percentage in weight in relation to the additive 1 is substantially below 1% for the etidronic acid and, in particular, substantially between 0.3% and 0.5%. The content in percentage in weight in relation to the additive 1 is practically between 0.5% and 5.5% for the water.

The use of an additive for hair dye, described above in a structural sense, is as follows.

In detail, usage requires that, at the moment of performing dyeing the operator prepares the dye by placing in a bowl and mixing with each other the oxidising agent, i.e. hydrogen peroxide, and precursors, and then adding the additive to the mixture of oxidising agent and precursors.

Alternatively, the operator first mixes the precursors with the additive in a bowl and only subsequently adds the hydrogen peroxide H₂O₂.

Once the mixing of the three components is complete, the dye is ready to be applied to the hair.

The invention comprises a new preparation method of a hair dye in which, as described above, the precursors, oxidising agent and additive for hair dye described above, are mixed together.

The invention achieves important advantages.

A first important advantage is represented by the fact that, thanks to the additive described above, the fumes of the hair dyes and, in particular, the ammonia fumes are greatly reduced.

In fact, the particular concentrations of citric and phosphoric acids allow the ammonia present in the dye to transform largely into water-soluble salts and, therefore, not to disperse into the air as a gas.

Another advantage is the fact that, thanks to the reduced presence of free ammonia in solution, the mixture of oxidising agent and precursors does not cause the development of irritating and malodorous gases and/or fumes in the atmosphere.

This advantage is particularly important for hairdressers and customers since the absence of such fumes makes it possible to have an odour-free environment.

Another advantage is that the additive does not alter the dyeing ability of the dye and does not therefore require either a longer-lasting application to the hair or greater frequency of application of the dye.

The cosmetic additive may be introduced directly into the hair dye in appropriate amounts during the industrial premixing of the various nuances, blocking the development of ammonia gas vapours in the atmosphere.

## Claims

1. Additive for hair dye **characterised in that** it comprises citric acid in a percentage in weight of 30% to 50%; phosphoric acid in a percentage in weight of 30% to 50%; and polyethylene glycol (PEG) in a percentage in weight of 20% to 30%.

2. Additive as claimed in claim 1, wherein said PEG is PEG 200.

3. Additive as claimed in claim 1 or 2, comprising dimethicone meadowfoamate.

4. Additive as claimed in one or more of the preceding claims, comprising dimethicone PEG-8 meadowfoamate.

5. Additive as claimed in one or more of the preceding claims, comprising propylene glycol.

6. Additive as claimed in one or more of the preceding claims, comprising etidronic acid.

7. Additive as claimed in one or more of the preceding claims, comprising water.

8. Additive as claimed in the previous claim, in which said water is de-ionised water.

9. Method of making a hair dye in which precursors, oxidant and a hair dye additive are mixed together, **characterised in that** said additive comprises citric acid in a percentage in weight of 30% to 50%; phosphoric acid in a percentage in weight of 30% to 50%; and PEG in a percentage in weight of 20% to 30%.

## Patentansprüche

1. Additiv für Haarfärbemittel, **dadurch gekennzeichnet, dass** es Zitronensäure in einem Gewichtsanteil zwischen im Wesentlichen 30 % und 50 %; Phosphorsäure in einem Gewichtsanteil zwischen im Wesentlichen 30 % und 50 %; und Polyethylenglycol (PEG) in einem Gewichtsanteil zwischen im Wesentlichen 20 % und 30 % umfasst.

2. Additiv nach Anspruch 1, bei dem das genannte PEG PEG 200 ist.

3. Additiv nach Anspruch 1 oder 2, das Dimethicone Meadowfoamate umfasst.

4. Additiv nach einem oder mehreren der vorangegangenen Ansprüche, das Dimethicone PEG-8 Meadowfoamate umfasst.

5. Additiv nach einem oder mehreren der vorangegangenen Ansprüche, das Propylenglycol umfasst.

6. Additiv nach einem oder mehreren der vorangegangenen Ansprüche, das Etidronsäure umfasst.

7. Additiv nach einem oder mehreren der vorangegangenen Ansprüche, das Wasser umfasst.

8. Additiv nach dem vorangegangenen Anspruch, bei dem das genannte Wasser demineralisiertes Wasser ist.

9. Verfahren zur Herstellung eines Haarfärbemittels, bei dem Vorläuferstoffe, Oxidationsmittel und ein Additiv für Haarfärbemittel miteinander gemischt werden, **dadurch gekennzeichnet, dass** das genannte Additiv Zitronensäure in einem Gewichtsanteil zwischen im Wesentlichen 30 % und 50 %; Phosphorsäure in einem Gewichtsanteil zwischen im Wesentlichen 30 % und 50 %; und PEG in einem Gewichtsanteil zwischen im Wesentlichen 20 % und 30 % umfasst.

## Revendications

1. Additif pour colorant capillaire **caractérisé en ce qu'**il comprend acide citrique en pourcentage en poids essentiellement compris entre 30 % et 50 % ; acide phosphorique en pourcentage en poids essentiellement compris entre 30 % et 50 % ; et polyéthylène glycol (PEG) en pourcentage en poids essentiellement compris entre 20 % et 30 %.

2. Additif selon la revendication 1 dans lequel ledit PEG est PEG200.

3. Additif selon la revendication 1 ou 2 comprenant dimethicone meadowfoamate.

4. Additif selon l'une ou plusieurs des revendications précédentes comprenant dimethicone PEG-8 meadowfoamate.

5. Additif selon l'une ou plusieurs des revendications précédentes comprenant poliéthylène glicol.

6. Additif selon l'une ou plusieurs des revendications précédentes comprenant acide étidronique.

7. Additif selon l'une ou plusieurs des revendications précédentes comprenant eau.

8. Additif selon la revendication précédente dans lequel ladite eau est déionisée.

9. Procédé pour la préparation d'un colorant capillaire dans lequel précurseurs, oxydant et un additif pour colorant capillaire sont réciproquement mélangés, **caractérisé en ce que** ledit additif comprend acide citrique en pourcentage en poids essentiellement compris entre 30 % et 50 % ; acide phosphorique en pourcentage en poids essentiellement compris entre 30 % et 50 % ; et PEG en pourcentage en poids essentiellement compris entre 20 % et 30 %.
